Europäisches Patentamt

European Patent Office

Office européen des brevets

(11) Publication number: **0 450 075 A1**

# EUROPEAN PATENT APPLICATION
## published in accordance with Art. 158(3) EPC

(21) Application number: 90900435.0

(22) Date of filing: 23.10.89

(86) International application number:
PCT/SU89/00270

(87) International publication number:
WO 91/05516 (02.05.91 91/10)

(51) Int. Cl.5: **A61B 17/60**

(43) Date of publication of application:
09.10.91 Bulletin 91/41

(84) Designated Contracting States:
AT BE CH DE FR GB IT LI SE

(71) Applicant: NAUCHNO-ISSLEDOVATELSKY
INSTITUT RADIOFIZIKI IMENI AKADEMIKA
A.A.RASPLETINA
NIIRF
Moscow, 123364(SU)

(72) Inventor: FURDJUK, Vasily Vasilievich
Mozhaiskoe sh., 29/2-199
Moscow, 121471(SU)

Inventor: KABANOV, Vladimir Efimovich
ul. Svobody, 2-33
Moscow, 123362(SU)
Inventor: REMIZOV, Boris Alexeevich
ul. Udaltsova, 16-155
Moscow, 117415(SU)
Inventor: SAMOTSVETOV, Anatoly Vasilievich
Khimkinsky bulvar, 14-1-16
Moscow, 123364(SU)

(74) Representative: Bibby, William Mark et al
Mathisen, Macara & co. The Coach House
6-8 Swakeleys Road
Ickenham Uxbridge UB10 8BZ(GB)

(54) **APPARATUS FOR OSTEOSYNTHESIS.**

(57) An apparatus for osteosynthesis comprises an oblong frame and at least four repositioning units. Each repositioning unit includes a slider (6) with two intersecting, longitudinal and transverse openings, a rod (15) mounted with the possibility of axial movement and fixation in the longitudinal opening of the slider (6), and a carrying element. Each carrying element consists of a thrust bushing (8) with an external thread. Through the axial opening of each thrust bushing (8) and longitudinal slots (5) in the longitudinal walls (3, 4) of the frame passes a fastening bolt (12).

FIG.4

FIG.13

Field of the Invention

The present invention relates to traumatology and orthopedics and more specifically to an osteosynthesis apparatus.

Prior Art

Present-day surgical practice utilizes rod-type apparatus for osteosynthesis of bone fragments, consisting of a supporting element, rods and fastening units for fixing the rods on the supporting element.

Known in the present state of the art is an osteosynthesis apparatus US, A, 4,570,625), comprising a supporting element appearing as a plain bar carrying a number of fastening units capable of longitudinal motion and fixation, each of said fastening units mounting a rod locked in place thereon and turned, with its self-tapping portion, into a respective bone fragments. Each of the bone fragments is fixed on two parallel arranged rods. The plain bar is provided with a device for longitudinal motion of the two rods fixed in the same bone fragment, whereby graduated distraction or compression of the bone fragments is ensured. However, the apparatus being discussed fails to provide graduated displacement (repositioning) of the bone fragments in a transvers plane, as well as in inclined planes, which places limitation on the field of application of the apparatus.

One more prior-art osteosynthesis apparatus (SU, A, 1,395,303) is known to comprise an oblong frame, each of two parallel longitudinal walls of which has a longitudinal recess, and at least four repositioning units. Each of the repositioning units incorporates a slider provided with two crossed holes, a longitudinal and a transverse, a carrier element mounted in the slider transverse hole with a possibility of longitudinal travel and fixation in the longitudinal recesses of the frame longitudinal walls, and a rod having a self-tapping and a threaded portion and fitted in the slider longitudinal hole with a possibility of axial displacement and fixation. Each of the carrier elements is shaped as a threaded bar. To provide a rigid construction the frame has the walls whose cross-sectional profile is shaped as an angle.

The apparatus described above is capable of imparting graduated displacement of the bone fragments along the orthogonal directions in a transverse plane in any position. However, said known apparatus fails to provide reposition of the bone fragments within six degrees of freedom. Besides, it is difficult to install the rods on the bone fragments in said apparatus, which is explained by a necessity of strict parallelism of the rods and difficulties encountered in fitting the threaded bars into the longitudinal frame recesses.

Disclosure of the Invention

It is a primary and essential object of the present invention to provide an osteosynthesis apparatus having such a construction arrangement of each repositioning unit that would considerably cut down the assembly time of the apparatus during surgery and the time for its removal after surgery, make it possible to replace some individual repositioning units without the frame removal, and extend the functional capabilities of the apparatus.

Said object is accomplished due to the fact that in an osteosynthesis apparatus, comprising an oblong frame, each of whose two parallel longitudinal walls having a longitudinal recess, and at least four repositioning units, each incorporating a slider provided with two crossed holes, i.e., a longitudinal and a transverse one, a carrier element installed in the slider transverse hole with a possibility of longitudinal travel and fixation in the longitudinal recesses of the frame longitudinal walls, and a rod having a self-tapping and a threaded portion, said rod being fitted in the slider longitudinal hole with a possibility of axial displacement and fixation, according to the invention, each of the carrier elements is shaped as a distance sleeve having a male thread and slots for a wrench, said slots being located nearby the sleeve ends adapted to interact with the inner surface of the frame lateral walls, while a tie bolt is passed through the bore of the distance sleeve and through the longitudinal recesses in the longitudinal frame walls.

It is expedient that the frame longitudinal walls be flat. Such a construction arrangement of the frame walls impart then the required elasticity in the plane of the frame arrangement. In addition, the frame flat walls make it possible to install or remove each of the repositioning units independently of the position assumed by the remaining units, which adds to the functional capabilities of the apparatus and cuts down its installation time.

It is expedient that the slider transverse hole be threaded and that the distance sleeve be turned into said threaded hole.

Such a construction arrangement simplifies the construction of the repositioning unit due to ruling out a locknut on the distance sleeve, as compared with the prototype, which results in a lower mass of the apparatus, reduces the quantity of its component parts and cuts down the installation time of the apparatus due to a shorter time required for fixing the repositioning unit in the frame.

It is expedient to provide the slider with a sliding nut whose body is expedient to be installed with a possibility of free rotation in the slider longitudinal hole, while the rod is reasonable to be turned, with its threaded portion, into the sliding nut. Such a construction feature adds to convenience in fitting the rods in the frame due to the provision of an external access to the fixing elements, unlike the case with the prototype, wherein one should manipulate a wrench in a restricted space between the frame and the patient's body, as well as reduces the installation time of the apparatus.

In order to extend the functional capabilities of the apparatus due to reposition of bone fragments along inclined directions in the frame transverse planes, it is expedient that the sliding nut be installed on the slider with a possibility of its angular displacement in the frame transverse plane.

Such an installation of the sliding nut also simplifies the process of rod installation, since the requirement of obligatory parallelism of the rods in bone fragments is no longer necessary. Besides, there are ensured reliable fixation of the rods in the frame regardless of their orientation with respect to the frame, as well as reduced time for installing the apparatus.

In order to extend the functional capabilities of the apparatus due to a possibility of fixing and repositioning the bone fragments of the femoral neck and of fixing a bone fragment with a single rod cantileverly held to the frame, it is expedient that the apparatus be provided with a femoral neck bone fragment fixing unit, comprising a body provided with two copplanar parallel transverse holes, a tie bolt being passed through each of said coplanar holes and through the longitudinal recesses in the frame longitudinal walls, and with one centre hole in which the sliding nut body is fitted with a possibility of free rotation, the axis of said centre hole being arranged at an angle of 120 to 130 degrees to the frame plane, and a rod turned into the sliding nut with its threaded portion.

It is expedient that a cylindrical recess having a length equal to 0.5 or 0.8 the length of the self-tapping rod portion and a depth equal to 0.2 or 0.6 the thread depth be provided on the self-tapping rod portion nearby the rod end.

Such a construction arrangement of the rod self-tapping portion adds to the rigidity of its fixation in the cortical layer of a tubular bone which is accounted for by compensation of enlargement of the initial hole upon turning the rod with a smaller-diameter thread due to turning larger-diameter thread root portion.

It is expedient to provide the apparatus with at least one emergency fixation unit, comprising two distance sleeves installed with a possibility of longitudinal travel and fixation and provided with a tie bolt passed through the bore of each of said distance sleeves and through the longitudinal recesses in the frame longitudinal walls, while each of said distance sleeves carries a slider with a collet grip which bear a plain stepped rod, said slider being capable of rotating with respect to said distance sleeve.

Such a construction feature extends the functional capabilities of the apparatus due to the provision of an emergency fixation of bone fragments under field conditions, which is necessary for patients' transportation to a hospital. Application of plain stepped rods makes it possible to expedite fixation of bone fragments, which in turn increases surgeon's labour productivity and ensures patients' admission to the hospital without additional injuring them while in transit.

It is favourable that the longitudinal recess in each of the frame longitudinal walls accommodate at least one bridge subdividing the longitudinal recess into portions.

Such a construction feature adds to the rigidity of the frame walls in a longitudinal plane and simplifies the construction of the apparatus due to dispensing with the elements for fixing the distance sleeves, such as C-washers, as well as makes it possible to attain higher fixing rigidity of bone fragments and faster installation of the apparatus.

It is expedient to provide the apparatus with a device for an adjustable longitudinal displacement of bone fragments, which would comprise a U-piece having longitudinal slots in its lateral walls, said slots being adapted for accommodating and fixing the tie bolts of at least two repositioning units, and a means for imparting longitudinal motion to the U-piece, while the heads of said tie bolts should be provided with threaded bosses.

Provision of such a device extends the functional capabilities of the apparatus due to an adjustable longitudinal displacement of bone fragments. Besides, the proposed construction of the device adds to convenience of carrying the apparatus by the patient due to a possibility of removing said device from the apparatus after carrying out a next adjusted displacement of bone fragments. Such a construction arrangement of the device for an adjustable longitudinal displacement of bone fragments enables it to be used in a variety of osteosynthesis apparatus.

To provide an adjustable longitudinal displacement of bone fragments and accelerate installation of the aforedescribed device on the frame, it is necessary that the U-piece longitudinal motion means be provided with a detachable grip adapted to be installed on the frame end wall, and with an adjusting screw which would be fitted in a threaded hole provided in the middle portion of the U-piece and be adapted to interact, through its end, with the detachable grip.

The osteosynthesis apparatus, according to the invention, provides for mutual repositioning of bone fragments within six degrees of fredom and makes it possible to avoid any types of dislocation of bone fragments, followed by their fixation till complete consolidation. The proposed apparatus ensures an adjustable displacement of bone fragments in three orthogonal directions, including graduated distraction and compression. The present apparatus is also applicable for osteosynthesis of the fragments of tubular bones in closed and open fractures of limbs, in fractures aggravated by osteomyelitis, in treatment of pseudarthrosis, in correcting osteotomies, in treatment of genicular or talocrural arthrosis, for reparation of bony defects with or without plastic surgery on soft tissues, for replanting the limb segments, or in treatment of the femoral neck fracture.

Summary of the Drawings

In what follows the present invention is illustrated by a detailed description of some specific exemplary embodiments thereof with reference to the accompanying drawings, wherein:

FIG. 1 is a view of an osteosynthesis apparatus, according to the invention;

FIG. 2 is top view of FIG. 1;

FIG. 3 is a longitudinal sectional view of a distance sleeve, according to the invention;

FIG. 4 is a section on the line IV-IV in FIG. 1:

FIG. 5 is a section taken on the line V-V in FIG. 1;

FIG. 6 is a general view of a rod;

FIG. 7 is a scaled-up view of the rod self-tapping portion, according to the invention;

FIG. 8 is a section taken along the line VIII-VIII in FIG. 7;

FIG. 9 is a general view of a slider with a sliding nut, according to the invention;

FIG. 10 is a view facing the arrow A in FIG. 9;

FIG. 11 is a first embodiment of the repositioning unit, wherein its slider carries the sliding nut, according to the invention;

FIG. 12 is a second embodiment of the repositioning unit, wherein its slider carries the sliding nut, according to the invention;

FIG. 13 illustrates an osteosynthesis apparatus, according to the invention, incorporating an emergency fixation unit;

FIG. 14 is a section taken along the line XIV-XIV in FIG. 13;

FIG. 15 is a section taken along the line XV-XV in FIG. 14;

FIG. 16 is a view facing the arrow B in FIG. 13;

FIG. 17 is a view of a femoral neck fragment fixing unit, according to the invention;

FIG. 18 is a view facing the arrow C in FIG. 17;

FIG. 19 is an embodiment of a longitudinal recess having a single bridge, according to the invention;

FIG. 20 shows a longitudinal recess of FIG. 19 provided with two bridges;

FIG. 21 illustrates an osteosynthesis apparatus, according to the invention, incorporating a device for adjustable longitudinal displacement of bone fragments;

FIG. 22 is a view facing the arrow D in FIG. 21;

FIG. 23 is a section taken along the line XXIII-XXII in FIG. 22;

FIG. 24 is a section taken along the line XXIV-XXIV in FIG. 23; and

FIG. 25 is a section taken along the line XXV-XXV in FIG. 22.

Preferred Embodiments of the Invention

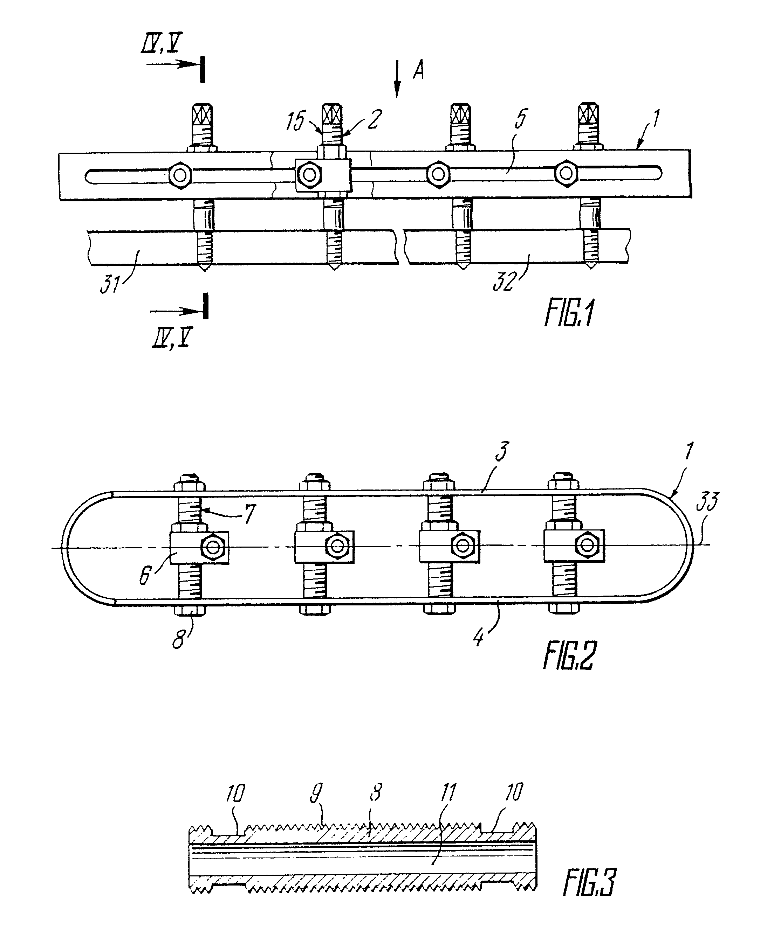

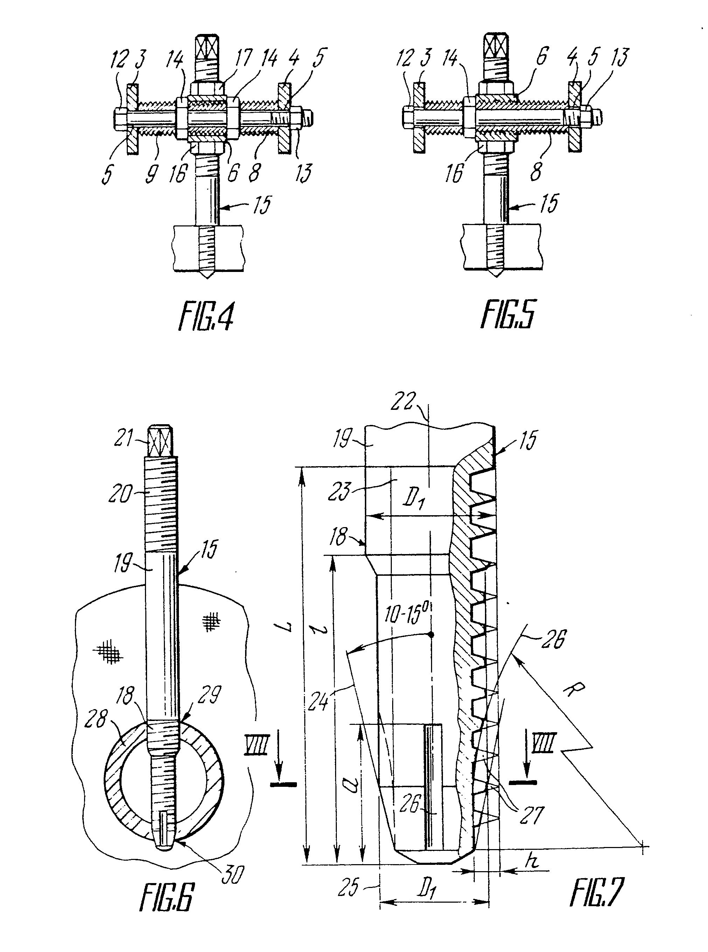

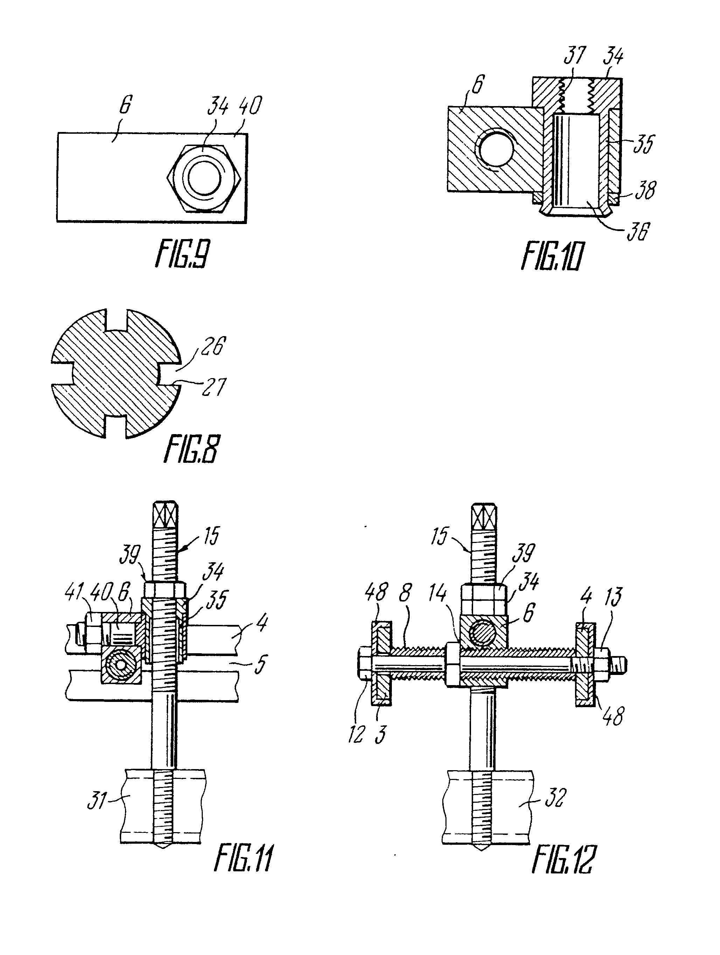

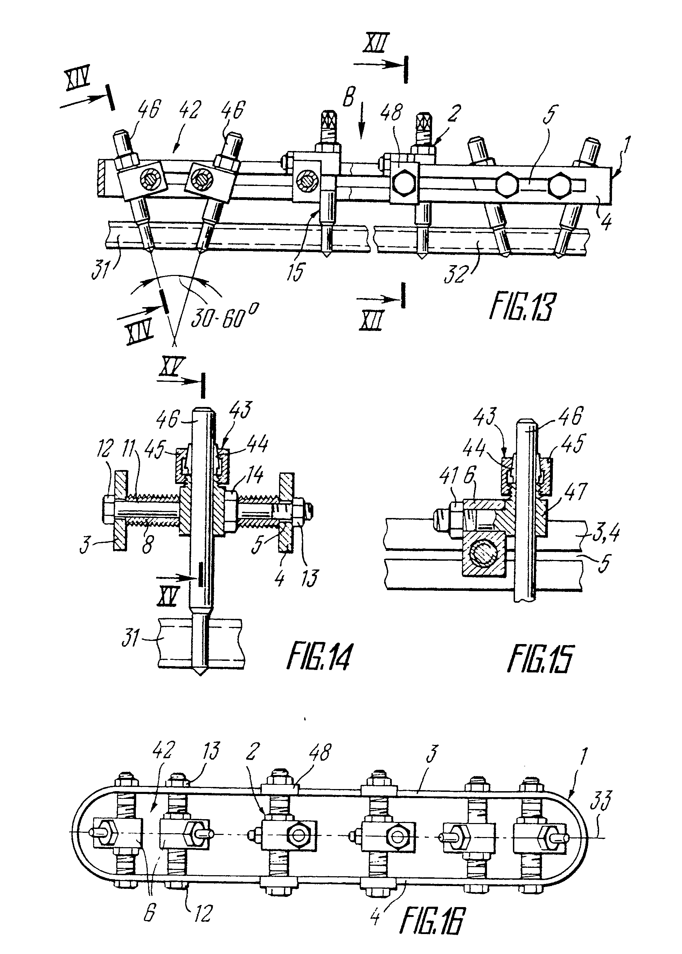

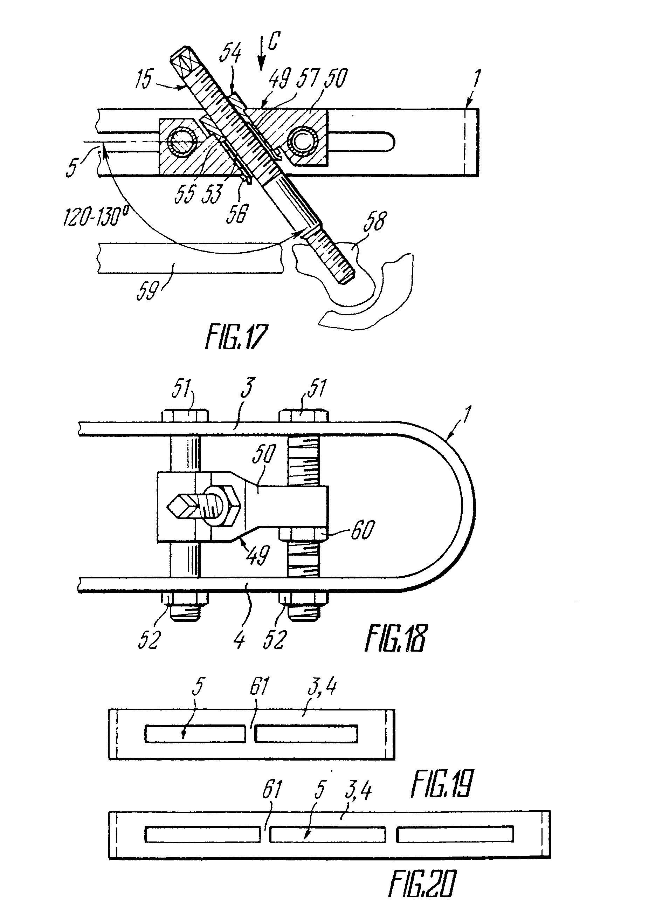

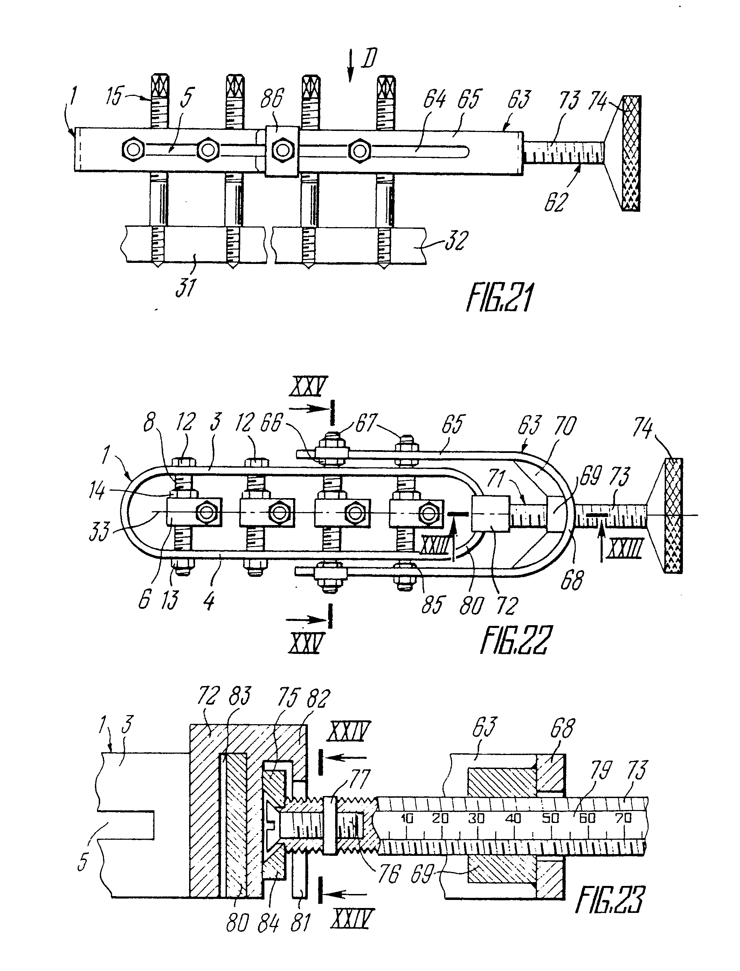

The osteosynthesis apparatus, according to the invention, comprises an oblong frame 1 (FIG. 1) and at least four repositioning units 2. Two longitudinal walls of the frame 1, viz., the wall 3 (FIG. 2) and the wall 4 are flat and parallel to each other. Each of the longitudinal walls 3 and 4 has a longitudinal recess 5 (FIG. 5).

Each of the repositioning units 2 comprises a slider 6 (FIG. 2) which has two crossed holes, a longitudinal and a transverse one. The transverse hole of the slider 6 accommodates a carrier element 7 mounted with a possibility of longitudinal travel and fixation in the longitudinal recesses 5 (FIG. 1) of the longitudinal walls 3, 4 (FIG. 2) of the frame 1. Each of the carrier elements 7 is shaped as a distance sleeve 8 (FIG.3) provided with a male thread 9 and slots 10 for wrench said slots being located nearby the sleeve ends adapted to interact with the inner surface of the longitudinal walls 3 (FIG. 4) and 4. A tie bolt 12 (FIG. 4) is passed through a bore 11 (FIG. 3) of each of the distance sleeves 8 and through the longitudinal recesses 5 (FIG. 4) of the frame 1 (FIG. 1), said tie bolt being locked with a nut 13 from outside.

The transverse hole in the slider 6 can be plain, in which case the slider 6 is locked in position on the distance sleeve 8 with two nuts 14. On the other hand, the transverse hole in the slider 6 can be threaded, which is the case shown in FIG. 5. In such a case the distance sleeve 8 is to be turned into said hole, while the slider 6 is locked in place on the distance sleeve 8 only with one nut 14, which provides a simpler construction (due to a lesser amount of the nuts 14), reduces the transverse dimensions of the apparatus (by the thickness of the nut 14) and hence cuts down the installation time of the apparatus.

A rod 15 is fitted in the longitudinal hole of each slider 6 with a possibility of axial displacement, said rod being locked in the slider 6 by means of two nuts 16, 17.

The rod 15 is shaped as a sequence of the following portions: a self-tapping portion 18 (FIG. 6), a plain portion 19, a threaded portion 20, and a tailpiece 21 having a polyhedral cross-section for wrench. The working end of the self-tapping portion 18 is blunted as, e.g. , a cone frustum, a rounded-off point, or some other shape with a reduced dimension along a axis 22 (FIG. 7). A thread 25 of the self-tapping portion 18 features a taper recess at an angle of 10 to 15 degrees to the axis 22 of the rod 15, and a cylindrical recess 25 for a depth of from 0.2 to 0.6 of the depth of the thread 23. The depth of the taper recess 24 equals the depth 'h' of the thread 23 at the end of the rod 15. The length 'l' of the cylindrical recess 25 equals 0.5 to 0.8 the length 'L' of the self-tapping portion 18, which has a number (3 to 4) of longitudinal slots 26 (FIG. 8) having the depth 'h' (FIG. 7) of the thread 23. The slots 26 can be of a square, triangular, trapezoidal, or some other cross-section, and with either a straight-line or arcuate bottom, the latter having a radius 'R' equal to the radius of a side milling cutter with the aid of which the slots have been milled. The slots 26 are longer than the taper recess 24, since it is necessary to provide formation of cutting faces 27 (FIG. 8) of the thread 23 (FIG. 7) both on the taper recess 24 and on the initial portion of the cylindrical recess 25. In individual instances the length 'a' of the slots 26 may be equal to the length 'L' of the self-tapping portion 18, which facilitates turning of the latter into the first cortical layer of a bone 28 (FIG. 6).

The apparatus is installed as follows.

Preliminarily a first hole 29 (FIG. 6) is drilled in the first cortical bone layer coaxially with a second hole which is drilled in the second cortical layer, both holes having a diameter $D = D_i - 2h$ (FIG. 7). When turning the rod 15, by means of a socket wrench, into the first hole 29, the bevelled faces 27 (FIG. 7) of turns of the thread 23 remove bone chips from the faces of the hole 29 (FIG. 6) to form a smaller-depth channel into which the rod 15 is adequately free to turn for a length 'a' (FIG. 7) of the cylindrical recess 25. Since the rod is selected beforehand having the length 'L' of its self-tapping portion 18 equal to the diameter of the bone 28 (FIG. 6), so on further turning of the rod 15 into the bone, the full-profile portion of the thread 23 (FIG. 7) approaches the first hole 29 simultaneously with approaching of the end of the rod 15 having the taper recess 24 (FIG. 7) to the second hole 30 (FIG. 6). Thus, a helical groove is formed in the second hole 30 (FIG. 6) simultaneously with deepening of the helical groove in the first hole 29. Inasmuch as the full thread 23 (FIG. 7) passes only 2 to 4 revolutions in the newly-formed deepened groove, the latter does not become loose, thus ensuring a tight and rigid fit of the rod 15 in the first hole 29 (FIG. 6). Accordingly, the end of the rod 15 is a tight and rigid fit in the second hole 30. The blunted end of the rod 15 goes beyond the second cortical layer of the bone 28 for a depth of 2 or 3 mm only, which inflicts less injury upon the soft tissues as compared with the known rods.

The process of installation of the rod 15 does not require a highly precision drilling of the holes 29 and 30 in the bone 28, since an adequately large depth 'h' (FIG. 7) of the thread 23 and the fact that the turns of the thread 23 fit snugly to the faces of the helical channel in the bone 28 (FIG. 6) across the full width thereof make it possible to provide fairly high rigidity of fixing of the rod 15 even when the diameters of the holes 29 and 30 gets somewhat enlarged due to radial runout in the course of drilling. It is not necessary in a given particular case to make use of reamers for an accurate establishing of the holes 29 and 30, which cuts down considerably the time for installation of the rod 15. It is due to the provision of, e.g., four longitudinal slots 26 (FIG. 8) spaced equidistantly along the periphery of the thread 23 (FIG. 7) and made adequately narrow that the strength of the remaining portion of the thread 23 (FIG. 7) not undercut at the root is ensured and a rigid fixing of the rod 15 in the bone 28 (FIG. 6) is attained even at the place of location of the slots 26 (FIG. 7).

Once the rods have been installed in bone fragments 31, 32 (FIG. 1), e.g., two rods in each of the bone fragments, the bottom nuts 16 (FIGS 4, 5) are to be screwed onto the back ends of the rods 15 and the sliders 6 are fitted thereonto after having preliminarily installed (or turned) the distance sleeves 8 with the nuts 14 into each of the sliders 6. Next the top nuts 17 are fitted onto each of the rods 15, whereupon the frame 1 (FIG. 1) is set parallel to the patient's body surface a distance of 1.5 to 2 cm apart so that the sliders 6 (FIG. 2) be arranged on an axis 33 of symmetry of the frame 1, and the tie bolts 11 are introduced from outside through the recess 5 (FIGS 4, 5) in the longitudinal wall 3, the distance sleeve 8 and the recess 5 in the longitudinal wall 4, after which the nut 13 is fitted onto the rod 15. Thereupon the nuts 13, 14, 17 are finally drawn tight to fix each of the distance sleeves 8 and of the rods 15 in position.

Whenever it is necessary to replace each of the rods 15, e.g., in the case of the rod 15 getting loose in the bone fragment 31 or 32 (FIG. 1) and a necessity to replace the rod 15 with that of a greater diameter, the nuts 13 (FIGS 4, 5) are unscrewed, the tie bolt 11 is extracted, the nut 17 is turned out and the slider 6 is removed along with the distance slleve 8. Then the rod 15 is turned out of the bone fragment 31 or 32 (FIG. 1), a fresh rod 15 is turned thereinto and fixed in position on the frame 1 in a reverse order.

To remove the frame 1, the nuts 13 (FIGS 4, 5) are unscrewed and the tie bolts 12 are removed, whereupon the frame 1 (FIG. 1) is taken out freely. Once the nuts 17 (FIGS 4, 5) have been turned out the sliders 6 along with the distance sleebes 8 are removed.

The construction of the apparatus described hereinabove provides for extended functional capabilities thereof as for a possibility of replacing some individual repositioning units 2 (FIG. 1) without removing the frame 1 but with the bone fragments 31, 32 remaining fixed in position with the aid of the remaining repositioning units 2.

Unlike the prototype the aforedescribed apparatus can be installed a single operator, which is of importance during surgery.

To provide higher convenience in installing the rods 15 in the frame 1 due to the provision of an outside access to the fixing elements (nuts), unlike the case with the prototype, wherein one should manipulate a wrench in a restricted space between the frame 1 and the patient's body, as well as to cut down the installation time of the apparatus the slider 6 (FIG. 9) is provided with a sliding nut 34 whose body 35 (FIG. 10) is mounted with a possibility of free rotation in the longitudinal hole of the slider 6. The sliding nut 34 has a nut provided with a female thread 37. The body 35 of the sliding nut 34 is expanded at one end and locked against falling out of the longitudinal hole of the slider 6 by a stop ring 38. The rod 15 (FIG. 11) is turned into the body 35 and locked with a nut 39 (FIG. 12) therein, though operation of the apparatus is possible without the use of the nut 39.

To extend the functional capabilities of the apparatus by repositioning the bone fragments along inclined directions in the transverse planes of the frame (FIG. 1), the sliding nut 34 (FIG. 9) is fitted on the slider 6 with a possibility of angular displacement in the transverse plane of the frame 1 (FIG. 1). To this end, a bracket is provided on the slider 6 (FIG. 10) with a possibility of swivelling in a transverse plane and fixing by means of a nut 41. The sliding nut 34 is installed on the bracket 40 with a possibility of free rotation. Locking of the sliding nut 34 and fitting the rod 15 (FIGS 11, 12) thereon are carried out in a way similar to the aforedescribed one.

Installing the sliding nut 34 with a possibility of angular displacement in a transverse plane simplifies the process of installation of the rods 15, since the requirement of the parallelism of the rods 15 in the bone fragments is no longer necessary. Besides, there is ensured reliable fixation of the rods 15 in the frame 1 (FIG. 1) whatever their orientation with respect to the frame 1 and the time for installation of the apparatus is curtailed.

The proposed osteosynthesis apparatus is provided with at least one emergency fixation unit 42 (FIG. 13). Provision of the unit 42 is indispensable for emergency fixation of the bone fragments 31, 32 under field conditions and for further patient's transportation to the hospital.

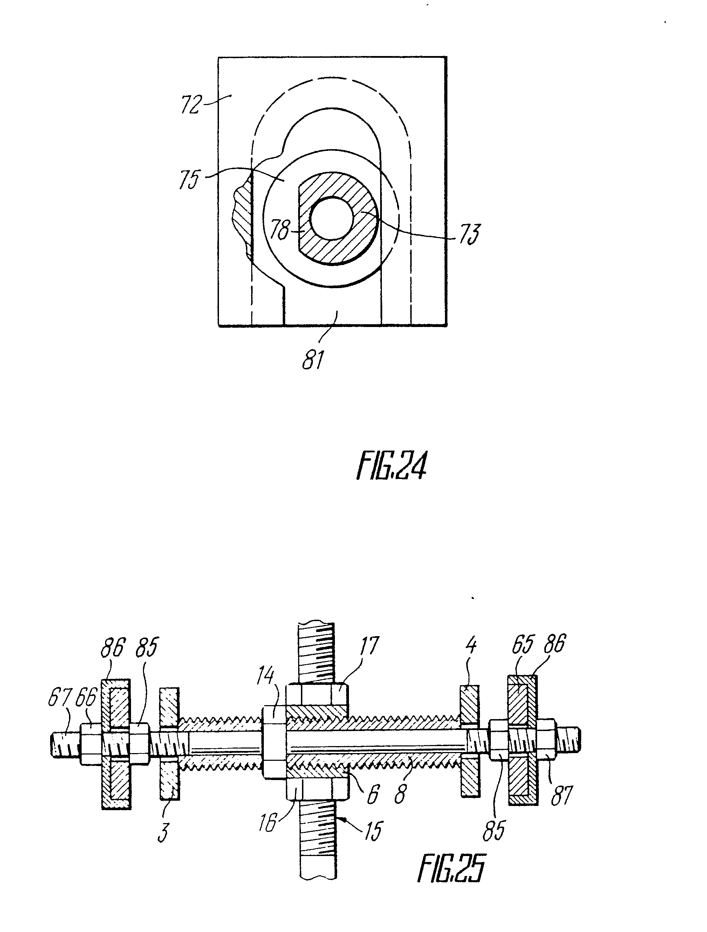

Each of the emergency fixation units 42 comprises two longitudinally movable distance sleeves 8 (FIG. 14), the tie bolt 12 being passed through the bore 11 of each of said sleeves and through the recesses 5 in the longitudinal walls 3, 4, and the slider 6 being mounted or each of said sleeves With a possibility of swivelling with respect to said sleeve. The slider 6 is provided with a collet grip 43 consisting of a collet 44 and a taper union nut 45. The collet grip 43 clamps a plain rod 46.

In each of the emergency fixation units 42 (FIG.13) the plain rods 46 are inclined towards each other at an angle of 30 to 60 degrees in a longitudinal plane of the frame 1. FIG. 13 presents the construction of the apparatus incorporating two emergency fixation units 42 and two repositioning units 2. Each of the emergency fixation units 42 establishes, together with part of the frame 1, a rigid truncated triangle which provides for reliable fixation of the bone fragment 31 or 32. An angle between the plain rods 46 below 30 degrees affects adversely the rigidity of the bone fragment fixation, while said angle exceeding 60 degrees involves a greater length of the frame 1. Each of the bone fragments 31, 32 should be fixed in place with at least one emergency fixation unit 42 provided with two plain rods 46.

In order to extend the operative capabilities of the apparatus by fixing those bone fragments in which the supporting holes have been drilled at different angles to a longitudinal plane of the frame 1, which is dictated by the configuration of the bone fragments and the drilling conditions in emergency situations, another embodiment of the construction of the emergency fixation unit 42 has been developed, according to which a bracket 47 is fitted into the hole in the slider 6 (FIG. 15) with a possibility of swivelling in a transverse plane and fixing by means of the nut 41. The bracket 47 mounts the collet 44 onto which the taper union nut 45 is screwed. The bearing portion of the plain rod 46 is locked in the collet grip 43. Provision of the bracket 47 makes it possible to fix bone fragments in cases where the supporting holes have been drilled therein at different angles to a longitudinal plane, which proves practically to be case nearly at all times.

The apparatus provided with the emergency fixation units 42 (FIG. 13) is installed as follows.

There are drilled the supporting holes in the bone fragments 31, 32 to install all the rods 15, 46. In this case the holes for installing the plain rods 46 are to be drilled with due allowance for a required angle (30 to 60 degrees), whereas the holes adapted to receive the rods 15 are to be reamed till a required diameter for their thread. Then the plain rods 46 are inserted into their respective supporting holes, and the rods 15 are turned-in with their self-tapping portion 18 (FIG. 6). Then the preassembled-together sliders 6 with the collet grips 43 (or with the sliders 6 carrying the brackets 47 (FIG. 15) with the collet grips 43) and with the distance sleeves 8 (FIG. 12) and the nuts 14 are fitted onto the plain rods 46 (FIG. 14) in a way described above, whereas screwed onto the rods 15 (FIG. 11) are the preassembled-together sliders 6 carrying the bracket 40 with the sliding nut 34. Once the distance sleeves 8 (FIGS 12, 14) have been adjusted for position with the respect to a longitudinal plane of the frame 1 (FIG. 13) and the sliders 6 (FIG. 11) have been adjusted for height on the rods 15 with respect to the surface of the patient's limb, the frame 1 (FIG. 13) is installed and the tie bolts 12 (FIG. 16) are passed through the longitudinal recesses 5 (FIG. 15) in the frame 1 and through the holes 11 (FIG. 3) in the distance sleeves 8, whereupon the nuts 13 (FIG. 16) are fitted in place in all the emergency fixation units 42. Next the sliders 6 are brought together manually in each of the emergency fixation unit 42 along the recesses 5 (FIG. 13) of the frame 1 so as to take up excess clearances between the supporting holes in the bone fragment 31 or 32 and the working portion of the plain rods 46. Once a tension force of 2 to 3 kgf enough to take up said excess clearances and to flatten the contact points on the bone fragment 31 or 32, the nuts 13 (FIG. 16) on the tie bolts are drawn tight and the emergency fixation units 42 are fixed in position in the frame 1 by drawing tight the taper nuts 45 (FIG. 1), the nuts 14 (FIG. 14) and the nuts 41 (FIG. 15). Thereupon the repositioning units are fixed in position in the frame 1 (FIG. 13) as described above.

To ensure against widening of the recesses 5 in the frame 1, use is made of C-washers, which prevents bending of the longitudinal members of each longitudinal wall 3, 4 (FIG. 16) of the frame 1 when drawing up the tie bolts 12 and in the course of carrying the apparatus by the patient.

Unfixing of the bone fragments 31, 32 (FIG. 13) and removal of the apparatus are carried out in the reversed order, i.e., first the nuts 13 (FIG. 16) are unscrewed, the tie bolts 12 are taken out, the frame 1 is removed, the taper nuts 45 (FIG. 14) are undone, the plain rods 46 are extracted, the rods 15 (FIG. 11) are turned out of the supporting holes and the repositioning units 2 (FIG. 13) are removed.

The proposed apparatus is equipped with a femoral neck bone fragment fixing unit 49 (FIG. 1). The aforesaid unit comprises a body 50 having two coplanar parallel holes, a tie bolt 51 being passed through each of said holes and through the recesses 5 in the longitudinal walls 3, 4 (FIG. 18) of the frame 1 and being fixed by a nut 52. The body 50 has a centre hole 53 (FIG. 17) whose axis makes an angle of from 120 to 130 degrees to the plane of the frame 1. A sliding nut 54 is mounted in the centre hole 53 with a possibility of free rotation, a body 55 of the nut 54 being expanded at one end and fixed against falling out of the centre hole 53 by means of a stop ring 56. The body 55 has a threaded hole 57 into which the rod 15 is turned.

The apparatus provided with the femoral neck bone fragment fixing device 49 is installed as follows. Supporting holes are drilled in bone fragments 58 and 59, whereupon the repositioning unit 2 (FIG. 1) are installed in the bone fragment 59 in a way similar to that described above. Then the rod 15 is turned into the bone fragment 58 (FIG. 17), and the sliding nut 54 is fitted onto the rod 15, said nut being fitted in the body 50 of the unit 49. Then the distance sleeves 8 and the longitudinal holes in the body 50 (FIG. 17) are brought in coplanarity by adjusting the position of the sliders 6 (FIG. 4) on the distance sleeves 8 and of the body 50 (FIG. 17) on the rod 15, whereupon the frame 1 is installed in position, the tie bolts 12 (FIG. 4) and 51 (FIG. 18) are fitted through the recesses 5 (FIG. 4) in the walls 3, 4 into the distance sleeves 8 and hence into the body 50 (FIG. 1) of the unit 49. Next the nuts 13 (FIG. 4) and, accordingly, the nuts 52 (FIG. 1) are drawn tight. The body 50 is locked against crosswise travel by a nut 60.

Provision of the unit 49 extends the functional capabilities of the present osteosynthesis apparatus due to a possibility of fixing and repositioning the bone fragments of the femoral neck and fixing the bone fragment with a single rod cantilevered to the frame.

In order to add to rigidity of the frame 1 in a longitudinal plane to simplify the construction of the apparatus in all the embodiments described hereinbefore, at least bridge 61 is provided in the longitudinal recess 5 (FIG. 19) of each of the longitudinal walls 3, 4, said bridge subdividing the longitudinal recess 5 into two portions. FIG. 20 illustrates the longitudinal recess 5 with two bridges 61. Provision of the bridges 61 makes it possible to dispense with fixing elements for the distance sleeves 8 (FIG. 12), such as C-washers 48, as well as to add to the rigidity of bone fragments and to accelerate installation of the apparatus.

The proposed osteosynthesis apparatus is provided with a device 62 (FIG. 21) for an adjustable longitudinal displacement of the bone fragments 31, 32. The device 62 comprises a U-piece 63 (FIG. 22) provided with longitudinal recesses 64 (FIG. 21) in its lateral walls 65, said recesses being adapted to accommodate and fix tie bolts 66 (FIG. 22) of at least two repositioning units 2, while the heads of the tie bolts 12 have threaded bosses 67. An end 68 of the U-piece 63 carries a sliding nut 69 and stiffening ribs 70, both said nuts and said ribs rigidly held to the U-piece 63. The device 62 is provided with a means 71 (FIG. 22) for imparting longitudinal motion to the U-piece 63, said means comprising a detachable grip 72 and an adjusting screw 73, which is mounted in the sliding nut 69 and is provided with a knob 74 and a thrust, washer 75 (FIG. 23), said washer being rotatably mounted at the end of the adjusting screw 73 by means of a screw 76 locked with a pin 77. The adjusting screw 73 has a flat 78 (FIG. 24) arranged lengthwise its shank, a metric scale 79 (FIG. 23) being provided on the flat 78. The grip 72 interlinking an end 80 of the frame 1 and the end of the adjusting screw 73 has an E-shaped cross-section and has a slot 81 in one of outer walls 82 thereof. When fitting the detachable grip 72 outside the frame 1 its end 80 engages a slot 83, while another slot 84 accommodates the thrust washer 75, and the slot 81 accommodates the end of the adjusting screw 73. The width of the slot 81 (FIG. 24) exceeds the diameter of the adjusting screw 73 but is smaller than the diameter of the thrust washer 75, which ensures against falling of the thrust washer 75 out when the adjusting screw 73 is being rotated and the U-piece 63 (FIG. 22) is longitudinally travelling. The U-piece 63 is so brought from the end 80 of the frame 1 that the recesses 64 (FIG. 21) in the wallS 65 slide over the ends of the tie bolts 66 (FIG. 22) and the ends of the threaded bosses 67 of the bolt heads. The U-piece 63 is fixed on the tie bolts 66 (FIG. 25) by means of undone locknuts 85, C-washers 86 and additional outer nuts 87.

The apparatus of the construction described above is installed as follows.

Prior to surgery one should turn the sliders 6 and the nut 14 onto the distance sleeves 8 so that the sliders 6 assume a middle position in the distance sleeves 8. Once the rods 15 have been fixed in the bone fragments 31, 32 during surgery, one nut 16 (FIG. 25, one slider 6 carrying the distance sleeve 8, and one nut 17 are fitted onto the back ends of the rods 15. Then the frame 1 (FIG. 21) is spaced 1 or 2 cm apart from the patient's body so that the rods 15 be arranged on the axis 33 (FIG. 22) of the frame 1, the transverse position of the sliders 6 is adjusted by rotating the distance sleeves 8, and the latter sleeves are locked in place by means of the tie bolts 12 and 66 fitted from outside. Once the frame 1 has been installed preliminarily the nuts 13, 14, 16 (FIG. 25), 17, 85 are drawn tight, thus ensuring rigid interconnection of the rods 15 and the frame 1 (FIG. 22).

To provide a longitudinal displacement of the bone fragments 31, 32 (FIG. 21) with respect to each other, the U-piece 63 is fitted onto the frame 1 from its end 80 (FIG. 22) in such a manner that the ends of the tie bolts 66 and of the threaded bosses 67 of the bolt heads should engage the recesses 64 (FIG. 21) in the walls 65 of said U-piece 63. Then the additional nuts 86 (FIG. 25) are fitted onto the tie bolts 66, whereupon the end of the adjusting screw 73 (FIG. 23) is brought to the frame end 80 and the detachable grip 72 is fitted from ouuside onto said end 80 and onto the thrust washer 75, the excess play is taken out by rotating the adjusting screw 73, the locknuts 85 (FIG. 25) are undone at the ends of the tie bolts 66, and said nuts 85 are drawn tight on the walls 65 of the U-piece 63 (FIG. 22), Thereby rigid holding of the other bone fragment 32 (FIG. 21) to the U-piece 63 is attained, as well as there is provided a free displacement of the distance sleeves 8 (FIG. 25) along the recesses 64 (FIG. 21). Next the bone fragments 31, 32 are displaced longitudinally by rotating the adjusting screw 73 either sense, and the amount of relative displacement of the bone fragments 31, 32 is read off the metric scale 79 (FIG. 23) on the adjusting screw 73, the end of the sliding nut 69 (FIG. 23) or the end 68 of the U-piece 63 serving as a fiducial point, since a difference between the readings taken before and after the displacement is essentially equal to the amount of displacement. Once the preset displacement of the bone fragments 31, 32 (FIG. 21) has been performed, the nuts 85 (FIG. 22) are drawn tight on the frame 1, thus releasing the walls 65 of the U-piece 63 and fixing the bone fragments 31, 32 (FIG. 21) in a new position on the frame 1. Then the grip 72 (FIG. 22) and the U-piece 63 are removed. All the procedures described above can be performed by a single operator.

Thus, the device 62 (FIG. 21) for adjustable longitudinal displacement of the bone fragments 31, 32 is installed on the frame 1 only for 3 to 5 minutes for simultaneous or multistep longitudinal displacement of said bone fragments, whereas all the rest of the operating time the frame 1 is free from said device 62. This enables the same device 62 to be utilized in a number of apparatus for extrafocal osteosynthesis, imposes no additional weight on the apparatus in the course of treatment and offers no inconvenience for the patient.

Industrial Applicability

The present apparatus is also applicable for osteosynthesis of the fragments of tubular bones in closed and open fractures of limbs, in fractures aggravated by osteomyelitis, in treatment of pseudarthrosis, in correcting osteotomies, in treatment of genicular or talocrural arthrosis, for reparation of bony defects with or without plastic surgery on soft tissues, for replanting the limb segments, or in treatment of the femoral neck fracture.

**Claims**

1. An osteosynthesis apparatus, comprising an oblong frame (1), each of whose two parallel longitudinal walls (3, 4) having a longitudinal recess (5), and at least four repositioning units (2), each incorporating a slider (6) provided with two crossed holes, i.e., a longitudinal and a transverse one, a carrier element (7) installed in the slider transverse hole with a possibility of longitudinal travel and fixation in the longitudinal recesses (5) of the frame (1) longitudinal walls (3, 4), and a rod (15) having a self-tapping portion (18) and a threaded portion (20), said rod being fitted in the slider longitudinal hole with a possibility of axial displacement and fixation, CHARACTERIZED in that each of the carrier elements (7) is shaped as a distance sleeve (8) having a male thread (9), while a tie bolt (12) is passed through a bore (11) of the distance sleeve (8) and through the longitudinal recesses in the longitudinal walls (3,4) of the frame (1).

2. The apparatus as claimed in Claim 1, CHARACTERIZED in that the frame longitudinal walls (3,4) are flat.

3. An apparatus as claimed in Claim 1, CHARACTERIZED in that the transverse hole of the slider (6) is threaded and the distance sleeve (8) is turned into said hole, the sleeve ends having slots (10) for a wrench.

4. An apparatus as claimed in Claim 1, CHARACTERIZED in that the slider (6) is provided with a sliding nut (34) whose body (35) is installed with a possibility of free rotation in the slider longitudinal hole, while the rod (15) is turned with its threaded portion (20) into the sliding nut (34).

5. An apparatus as claimed in Claim 4, CHARACTERIZED in that the sliding nut (34) is installed on the slider (6) with a possibility of angular displacement in the frame (1) transverse plane.

6. An apparatus as claimed in Claim 1, CHARACTERIZED in that provision is therein made for a femoral neck bone fragment fixing unit (49), comprising a body (50) provided with two coplanar parallel transverse holes, a tie bolt (51) being passed through each of said coplanar holes and through the longitudinal recesses (5) in the frame longitudinal walls (3,4), with one centre hole (53), wherein body a (55) of a sliding nut (54) is fitted with a possibility of free rotation, the axis of said centre hole being arranged at an angle of 120 to 130 deggrees to the frame (1) plane, and the rod (15) turned into the sliding nut (54) with its threaded portion (20).

7. An apparatus as claimed in Claims 4 and 6, CHARACTERIZED in that a cylindrical recess (25) is provided on the self-tapping rod portion (18) nearby the rod end, said recess having a length ($\ell$) equal to 0.5 or 0.8 the length (L) of the self-tapping rod portion (18) and a depth, equal to 0.2 or 0.6 a depth (h) of the thread (23).

8. An apparatus as claimed in Claim 1, CHARACTERIZED in that provision is therein made for at least one energency fixation unit (42), comprising two distance sleeves (8) installed with a possibility of longitudinal travel and fixation and furnished with the tie bolt (12) passed through the bore (11) of each of said distance sleeves (8) and through the longitudinal recesses (5) in the frame longitudinal walls (3,4), while each of said distance sleeves (8) carries the slider (6) with a collet grip (43) which bears a plain stepped rod (46), said slider being capable of rotating with respect to the distance sleeve (8).

9. An apparatus as claimed in Claim 1, CHARACTERIZED in that the longitudinal recess (5) in each of the frame longitudinal wall (3,4) has at least one bridge (61) that subdivides the longitudinal recess (5), into portions.

10. An apparatus as claimed in Claim 1, CHARACTERIZED in that provision is therein made for a device (62) for an adjustable longitudinal displacement of bone fragments (31, 32) said device comprising a U-piece (63) having longitudinal slots (64) in its lateral walls (65), said slots being adapted to accommodate and fix tie bolts (66) of at least two repositioning units (2), and a means (71) for imparting longitudinal motion to said U-piece (63), while the heads of said tie bolts (66) are provided with threaded bosses (67).

11. An apparatus as claimed in Claim 10, CHARACTERIZED in that the means (71) for imparting longitudinal motion to the U-piece (63) comprises a detachable grip (72) adapted to be installed on the frame end wall (68) and an adjusting screw (73), which is fitted in a threaded hole provided in the U-piece (63) middle portion and is adapted to interact, through its end, with the detachable grip (72).

FIG.1

FIG.2

FIG.3

EP 0 450 075 A1

FIG.4

FIG.5

FIG.6

FIG.7

13

FIG.9

FIG.10

FIG.8

FIG.11

FIG.12

FIG.13

FIG.14

FIG.15

FIG.16

FIG.17

FIG.18

FIG.19

FIG.20

16

FIG.21

FIG.22

FIG.23

FIG.24

FIG.25

# INTERNATIONAL SEARCH REPORT

International Application No  PCT/SU89/00270

## I. CLASSIFICATION OF SUBJECT MATTER (if several classification symbols apply, indicate all) 6

According to International Patent Classification (IPC) or to both National Classification and IPC

Int.Cl.5          A61B 17/60

## II. FIELDS SEARCHED

### Minimum Documentation Searched 7

| Classification System | Classification Symbols |
|---|---|
| Int.Cl.4 | A61B 17/56, 17/58, 17/60 |

### Documentation Searched other than Minimum Documentation to the Extent that such Documents are Included in the Fields Searched 8

## III. DOCUMENTS CONSIDERED TO BE RELEVANT 9

| Category * | Citation of Document, 11 with indication, where appropriate, of the relevant passages 12 | Relevant to Claim No. 13 |
|---|---|---|
| A | SU, AI, 1395303 (A. N. KOSTJUK) 15 May 1988 (15.05.88), see the claims and figure 2 (cited in the description) | 1,2,5 |
| A | SU, AI, 1475630 ( A. N. KOSTJUK) 30 April 1989(30.04.89),see claims, figure 1 | 3,4,9 |
| A | SU, AI, 330857 (A. M. VINOHUR) 12 April 1972 (12.04.72) see the claims and figure 1 | 10,11 |
| A | SU, AI, 1284533 (KHARKOVSKY NAUCHNO-ISSLEDOVATELSKY INSTITUT ASTOPEDII I TRAVMATOLOGII IM. PROF. M.I. Sitenko) 23 January 1987 (23.01.87) see the claims and figure 1 | 7 |
| A | US, A, 2251209 (OTTO STADER) 29 July 1941 (29.07.41) see the claims and figure 6 | 6,8 |
| A | US, A, 2391537 (R. ANDERSON) 25 December 1945 (25.12.45) see the claims and figure 1 | |

* Special categories of cited documents: 10

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art.

"&" document member of the same patent family

## IV. CERTIFICATION

| Date of the Actual Completion of the International Search | Date of Mailing of this International Search Report |
|---|---|
| 25 January 1990 (25.01.90) | 14 March 1990 (25.03.90) |

| International Searching Authority | Signature of Authorized Officer |
|---|---|
| ISA/SU | |

Form PCT/ISA/210 (second sheet) (January 1985)